# EUROPEAN PATENT SPECIFICATION

(11) **EP 4 578 864 B1**
(45) Date of publication and mention of the grant of the patent: **07.10.2026**
(21) Application number: 23857309.1
(22) Date of filing: 21.08.2023
(51) Int. Cl.: C07F 7/12, C07H 23/00, C07H 1/00, C07F 7/18, C07F 7/08

(54) **TRITYL-BASED ALCOHOL PROTECTING GROUP**
TRITYLBASIERTE ALKOHOLSCHUTZGRUPPE
GROUPE PROTECTEUR D'ALCOOL À BASE DE TRITYLE

(30) Priority: 22.08.2022 JP 2022131789
(43) Date of publication of application: 02.07.2025
(73) Proprietor: SynCrest Inc., Fujisawa-shi, Kanagawa 251-8555 (JP)
(72) Inventor: XU, Pengyu, Fujisawa-shi, Kanagawa 251-8555 (JP); YONEYAMA, Shin, Fujisawa-shi, Kanagawa 251-8555 (JP)
(74) Representative: TBK
(86) International application number: PCT/JP2023/029912
(87) International publication number: WO 2024/043198

(56) References cited:
- WO-A1-2021/020561
- JIANG XING ET AL: "Crystal Fluidity Reflected by Fast Rotational Motion at the Core, Branches, and Peripheral Aromatic Groups of a Dendrimeric Molecular Rotor", JOURNAL OF THE AMERICAN CHEMICAL SOCIETY, vol. 138, no. 13, 25 March 2016 (2016-03-25), pages 4650 - 4656, XP093324023, ISSN: 0002-7863, DOI: 10.1021/jacs.6b01398
- MYAKINA N I: "Synthesis of symmetric radicals of triphenylmethyl derivatives containing multiple and single bonds", IZVESTIYA AKADEMII NAUK / OTDELENIE KHIMICHESKAYA NAUK, vol. 6, 1 January 1973 (1973-01-01), RU, pages 1405 - 1407, XP093324019, ISSN: 0002-3353
- SEKINE MITSUO: "Development of new protective groups for sugar hydroxyl groups used in nucleic acid synthesis", ORGANIC SYNTHETIC CHEMISTRY, vol. 45, no. 10, 1 January 1987 (1987-01-01), pages 930 - 943, XP093144467
- SHARMA G. V. M., PRASAD T. RAJENDRA, RAKESH, SRINIVAS B.: "p ‐Methoxy Diphenylmethanol (MDPM), p ‐Phenyl Diphenylmethanol (PDPM), and p ‐Phenylphenyl Diphenylmethanol (PPDPM)—Protecting Groups for Alcohols—Protection and Deprotection", SYNTHETIC COMMUNICATIONS, TAYLOR & FRANCIS INC., US, vol. 34, no. 5, 31 December 2004 (2004-12-31), US , pages 941 - 950, XP093144470, ISSN: 0039-7911, DOI: 10.1081/SCC-120028367
- OSSWALD STEFFEN, BREIMAIER STEFANIE, LINSEIS MICHAEL, WINTER RAINER F.: "Polyelectrochromic Vinyl Ruthenium-Modified Tritylium Dyes", ORGANOMETALLICS, AMERICAN CHEMICAL SOCIETY, vol. 36, no. 10, 22 May 2017 (2017-05-22), pages 1993 - 2003, XP093144473, ISSN: 0276-7333, DOI: 10.1021/acs.organomet.7b00194
- HIROYUKI YAMAKOSHI, KOSUKE DODO, MASAYA OKADA, JUN ANDO, ALMAR PALONPON, KATSUMASA FUJITA, SATOSHI KAWATA, MIKIKO SODEOKA: "Imaging of EdU, an Alkyne-Tagged Cell Proliferation Probe, by Raman Microscopy", JOURNAL OF THE AMERICAN CHEMICAL SOCIETY, AMERICAN CHEMICAL SOCIETY, vol. 133, no. 16, 27 April 2011 (2011-04-27), pages 6102 - 6105, XP055232088, ISSN: 0002-7863, DOI: 10.1021/ja108404p

## Description

### Technical Field

The present invention relates to a trityl-based alcohol protecting group.

### Background Art

NPL 1 and NPL 2 disclose a method for synthesizing a nucleic acid using a 4,4'-dimethoxytrityl group (a DMTr protecting group).

WO 2021/020561 A1 discloses a primer, an apparatus for producing double-stranded dna using same, and a method for producing double-stranded dna. In particular, a primer is provided which is used in amplifying a nucleic acid and has a structure represented by Formula (1). (wherein A1 represents -S-, -S-S-, or -Se-, B represents a base, and R1 represents a degradable protecting group. * means a bond to the sugar of an adjacent nucleotide.)

### Citation List

### Non-patent Literature

NPL 1: Tetrahedron Letters, 22, 1859-1862, 1981
NPL 2: J. Am. Chem. Soc., 85, 3821-3827, 1963

### Summary of Invention

### Technical Problem

An object of the present invention is to provide a novel trityl-based alcohol protecting group that is useful in synthesizing nucleic acids.

### Solution to Problem

The present inventors have conducted extensive research to achieve the above object. As a result, the present inventors found that by introducing at least one triple bond into a protecting group, such as a 4,4'-dimethoxytrityl group (a DMTr protecting group), a novel trityl-based alcohol protecting group that is useful in synthesizing nucleic acids can be produced.

The present inventors conducted further research based on this finding and have accomplished the present invention.

Specifically, the present invention includes the following trityl-based alcohol protecting groups.

The present invention provides a compound as defined in the enclosed independent claim 1. Further beneficial embodiments of the present invention are described in the enclosed dependent claims.

In detail, the compound of the present invention, is as defined in claim 1, and thus, is a compound represented by the following formula (1): 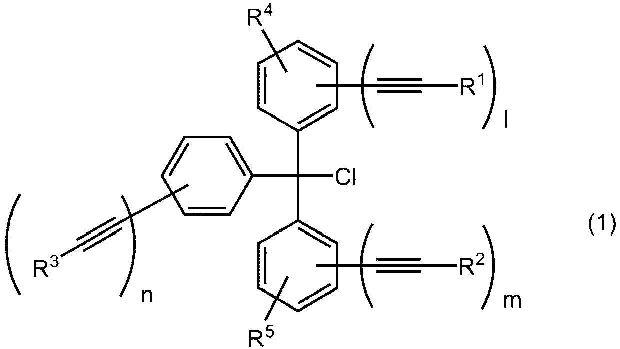 (wherein
R¹, R², and R³ are same or different, and each represents a hydrogen atom, an alkyl group, a trimethylsilyl group (TMS, -Si(CH₃)₃), an acetyl group (Ac), a benzoyl group (Bz), an ester group, an alkyl ester group, an aryl group, an alkyl alcohol group, a nitrile group (CN), a group represented by 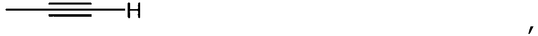 or a group represented by 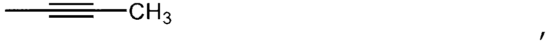
wherein l, m, and n each represent an integer of 0, 1, or 2 (provided that l, m, and n are not 0 at the same time), and
R⁴ and R⁵ are the same or different, and each represents an electron-donating group).

As a trityl-based alcohol protecting group, the compound represented by formula (1) of the present invention is excellent in controlling the ease of deprotection. The compound represented by formula (1) of the present invention enables improvement of a substituent, such as a DMTr protecting group, and adjustment of the deprotection conditions to thereby increase the yield of the nucleic acid synthesized. Based on the triple bond introduced into the trityl-based alcohol protecting group, the compound represented by formula (1) of the present invention can change cationic properties of the trityl moiety and allows the protecting group to be readily removed for deprotection.

The triple bond introduced into the compound represented by formula (1) of the present invention in the form of a trityl-based alcohol protecting group can be detected by Raman spectroscopy. Based on the trityl-based alcohol protecting group, the compound represented by formula (1) of the present invention allows for detection of quantifiable peaks in the silent region and enables quantification of the protecting group without being disturbed by other impurities and is excellent in tracking various reactions.

### Advantageous Effects of Invention

The present invention can newly provide a trityl-based alcohol protecting group that is useful in synthesizing nucleic acids.

### Description of Embodiment

The present invention is described in detail below.

In the present specification, the terms "comprise" and "contain" encompass the concepts of comprising, consisting essentially of, and consisting of.

In the present specification, the numerical range indicated by "A to B" means A or more and B or less, unless otherwise specified.

### [1] Trityl-based Alcohol Protecting Group

The trityl-based alcohol protecting group of the present invention is a compound represented by the following formula (1):

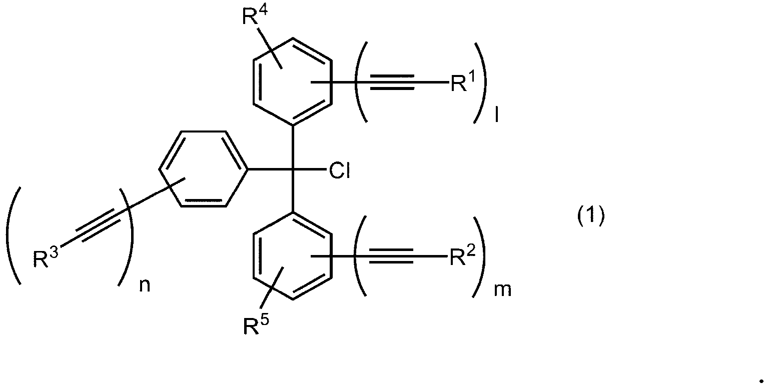

In formula (1), R¹, R², and R³ are same or different, and each represents a hydrogen atom, an alkyl group, a trimethylsilyl group, an acetyl group, a benzoyl group, an ester group, an alkyl ester group, an aryl group, an alkyl alcohol group, a nitrile group, a group represented by 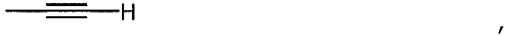 or a group represented by formula (6)

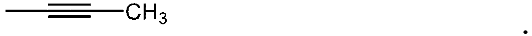

In formula (1), l, m, and n each represent an integer of 0, 1, or 2, provided that l, m, and n are not 0 at the same time.

In formula (1), R⁴ and R⁵ may be same or different, and each represents an electron-donating group.

### R¹, R², and R³

In formula (1), R¹, R², and R³ are same or different, and each represents a hydrogen atom (H), an alkyl group, a trimethylsilyl group (TMS, -Si(CH₃)₃), an acetyl group (Ac), a benzoyl group (Bz), an ester group, an alkyl ester group, an aryl group, an alkyl alcohol group, a nitrile group (CN), a group represented by 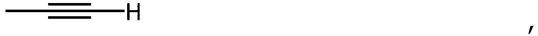 or a group represented by

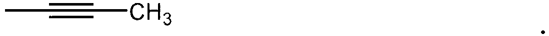

The alkyl group is preferably, for example, a methyl group (-CH₃, Me), an ethyl group (Et), an n-butyl group (n-Bu), or a t-butyl group (t-Bu).

The ester group is preferably, for example, a methyl ester group (COOMe) or an ethyl ester group (COOEt).

The alkyl ester group is preferably, for example, a 3-carboxypropyl group ((CH₂)₃COOH) or a 3-methyloxycarbonylpropyl group ((CH₂)₃COOMe).

The aryl group is preferably, for example, a phenyl group (Ph), a 4-methyloxycarbonylbenzene group (4-COOMe-C₆H₄), a 4-formylbenzene group (4-CHO-C₆H₄), a 4-methylhydroxybenzene group (4-CH₂OH-C₄H₆), or a 3,5-dimethoxybenzene group (3,5-OMe-C₆H₄)

The alkyl alcohol group (e.g., CH₂OH, (CH₂)₂OH, (CH₂)₃OH), CH₂OCO₂(4-NO₂-C₄H₆), or (CH₂)₃OCO₂(4-NO₂-C₄H₆) is preferably, for example, a methyl hydroxyl group (CH₂OH), an ethyl hydroxyl group ((CH₂)₂OH), a propyl hydroxyl group ((CH₂)₃OH), a methyl(4-nitrobenzo)oxycarbonyl group (CH₂OCO₂(4-NO₂-C₄H₆)), or a propyl(4-nitrobenzo)oxycarbonyl group ((CH₂)₃OCO₂(4-NO₂-C₄H₆).

In formula (1), the substituent of a triple bond (alkyne) including R¹: 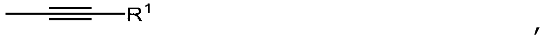
the substituent of a triple bond (alkyne) including R²: 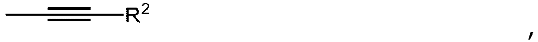 and
the substituent of a triple bond (alkyne) including R³: 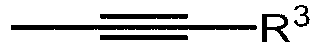 may be bonded at any of the ortho-, meta-, or para-positions.

In formula (1), the substituent of a triple bond including R¹, the substituent of a triple bond including R², and the substituent of a triple bond including R³ are each bonded, for example, at a para-position with respect to the carbon atom of the benzene ring bonded to the central carbon atom (the "-C" in "-C(C₆H₅)₃") of the trityl group (-C(C₆H₅)₃); and
l, m, and n each represent an integer of 0, 1, or 2,
provided that l, m, and n are not 0 at the same time,
l is the number of substituents in the triple bond including R¹ (alkyne),
m is the number of substituents in the triple bond including R² (alkyne), and
n is the number of substituents in the triple bond including R³ (alkyne).

In formula (1), at least one of R¹, R², and R³ is preferably a trimethylsilyl group.

### R⁴ and R⁵

In formula (1), R⁴ and R⁵ may be same or different, and each represents an electron-donating group.

The electron-donating group is as defined in claim 1 and, thus, is
a linear or branched alkyl group, such as methyl, ethyl, n-propyl, isopropyl, n-butyl, isobutyl, or tert-butyl;
a cycloalkyl group, such as cyclopropyl, cyclobutyl, or cyclopentyl;
an alkenyl group, such as vinyl, allyl, or 1-propenyl;
a cycloalkenyl group, such as 1-cyclopentenyl or 1-cyclohexenyl;
an aryl group, such as phenyl;
an aralkyl group, such as benzyl or phenethyl;
an alkoxy group, such as methoxy, ethoxy, n-propoxy, iso-propoxy, n-butoxy, or tert-butoxy;
an aryloxy group, such as phenyloxy;
a hydroxy group;
an amino group;
an amino group having one or two alkyl groups, such as methylamino, ethylamino, n-propylamino, dimethylamino, or diethylamino;
an amino group having one or two aryl groups, such as phenylamino; or a hydroxyamino group.

The electron-donating group is as defined in claim 1, and, thus, can also be an alkyl sulfide group, such as a methyl sulfide group (methylthio group), an ethyl sulfide group, an n-propyl sulfide group, an isopropyl sulfide group, an n-butyl sulfide group, an isobutyl sulfide group, or a tert-butyl sulfide group.

The electron-donating group is more preferably, for example, a linear or branched alkyl group, an alkoxy group, a hydroxy group, an amino group, or an amino group having one or two alkyl group, even more preferably an alkoxy group, a hydroxy group, or an amino group, and particularly preferably an alkoxy group having 1 to 4 carbon atoms.

The electron-donating group is preferably an alkoxy group, such as methoxy (-OMe), ethoxy (-OEt), n-propoxy (-OnPr), isopropoxy (-OiPr), n-butoxy (-OnBu), or tert-butoxy (-OtBu).

The electron-donating group is particularly preferably a linear or branched alkyl group, such as methyl, ethyl, n-propyl, isopropyl, n-butyl, isobutyl, or tert-butyl.

The electron-donating group is particularly preferably an alkylsulfide group, such as a methylsulfide group (e.g., a methylthio group, -S-Me, -S-CH₃) or an ethylsulfide group (-S-Et).

In formula (1), R⁴ and R⁵ may be each bonded at any of the ortho-, meta-, or para-positions.

In formula (1), R⁴ and R⁵ are each preferably, for example, bonded at a para-position with respect to the carbon atom of a benzene ring bonded to the central carbon atom ("-C" in "-C(C₆H₅)₃") of the trityl group (-C(C₆H₅)₃).

The trityl-based alcohol protecting group of the present invention is a protecting group, such as a 4,4'-dimethoxytrityl group (a DMTr protecting group), that has at least one triple bond introduced into the protecting group. The triple bond introduced into the trityl-based alcohol protecting group conjugates with a DMTr protecting group or like protecting group to thereby change cationic properties of the trityl moiety of the DMTr protecting group. Due to this feature, the trityl-based alcohol protecting group of the present invention can control the ease of removal of the protecting group from a target nucleic acid.

The DMTr protecting group or like protecting group of the trityl-based alcohol protecting group changes in stability of the cation. For example, under weak acidic conditions, the protecting group forms a trityl-based alcohol protecting group that can be easily or instantaneously removed.

The triple bonds introduced into the trityl-based alcohol protecting group of the present invention produce quantifiable peaks in the silent region in Raman spectroscopy. Due to this feature, the trityl-based alcohol protecting group of the present invention allows for quantitative determination of the protecting group without being interfered with by other impurities.

The trityl-based alcohol protecting group of the present invention is useful in producing nucleic acids.

### Examples

Embodiments of the present invention are described in more detail below based on Examples.

The present invention is not limited to these Examples.

### Examples

### [1] Production 1 of trityl-based alcohol protecting group (Scheme 1)

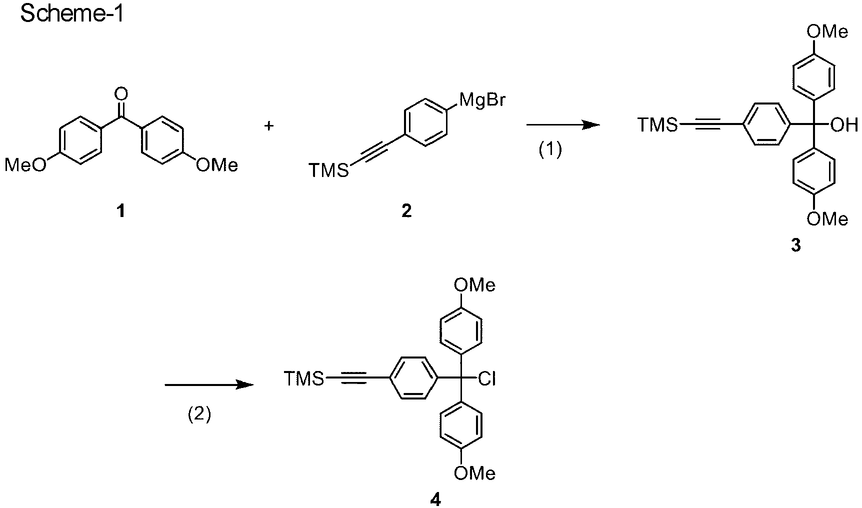

### (1-1) Production of Compound 3 in Scheme 1

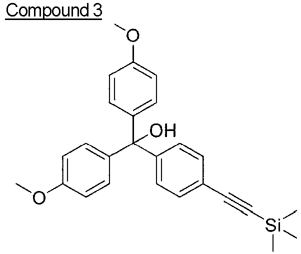

Under an argon atmosphere, magnesium powder (720 mg, 29.6 mmol) and iodine (2 items) were placed in a 100 mL roundbottom flask, and 23 mL of tetrahydrofuran (THF) was added. The resulting mixture was stirred at room temperature. A solution (8.0 mL) of 4-(bromophenylethynyl)trimethylsilane (a precursor of Compound 2 in Scheme 1) (5.00 g, 19.7 mmol) in THF was added dropwise thereto and the resulting mixture was stirred at room temperature for 2 hours to prepare a Grignard reagent of 4-(bromophenylethynyl)trimethylsilane (Compound 2 in Scheme 1).

4,4'-Dimethoxybenzophenone (Compound 1 in Scheme 1) (4.29 g, 17.7 mmol) was added to this reaction liquid, and the resulting mixture was stirred at room temperature for 3 days. After completion of the reaction was confirmed, an aqueous ammonium chloride solution was added to the reaction liquid, and the resulting mixture was extracted with ethyl acetate. The organic layer was dried over magnesium sulfide and concentrated.

The residue was purified by silica gel column chromatography (hexane/ethyl acetate = 19/1 → 9/1) to obtain the target compound (Compound 3 in Scheme 1) (4.63 g, 11.1 mmol) as a white solid.

¹H-NMR (CDCl₃): δ 7.42-7.38 (m, 2H), 7.25-7.21 (m, 2H), 7.16-7.11 (m, 4H), 6.85-6.80 (m, 4H), 3.80 (s, 6H), 2.66 (s, 1H), 0.24 (s, 9H)
¹³C-NMR (CDCl₃): δ 158.8, 147.6, 139.0, 131.5, 129.1, 127.6, 121.8, 113.3, 104.9, 94.3, 81.3, 55.3, 0.0

### (1-2) Production of Compound 4 (trityl-based alcohol protecting group) in Scheme 1

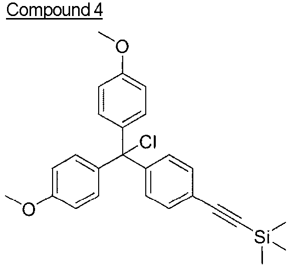

Thionyl chloride (0.17 mL, 2.4 mmol) and N,N-dimethylformamide (DMF) (1 drop) were added to 4-trimethylsilylethynyl-4',4"-dimethoxyltrityl alcohol (Compound 3 in Scheme 1) (0.20 g, 0.48 mmol), and the resulting mixture was stirred at 45°C for 1.5 hours.

The stirred mixture was then concentrated and the residue of the target compound was obtained as a red solid (Compound 4 (trityl-based alcohol protecting group) in Scheme 1).

¹H-NMR (CDCl₃): δ 7.41-7.37 (m, 2H), 7.22-7.18 (m, 2H), 7.15-7.10 (m, 4H), 6.84-6.79 (m, 4H), 3.82 (s, 6H), 0.25 (s, 9H)
¹³C-NMR (CDCl₃): δ 159.3, 146.1, 137.4, 131.4, 131.1, 129.7, 122.7, 113.2, 113.1, 104.7, 95.3, 82.4, 55.5, 0.1

### (1-3) Protection of nucleic acid by trityl-based alcohol protecting group (Scheme 2)

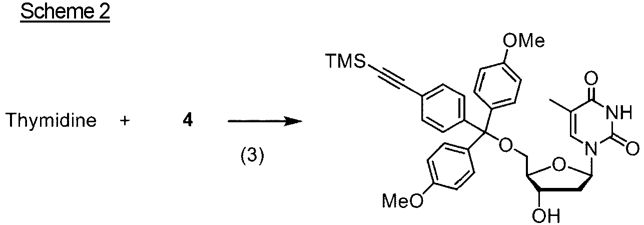

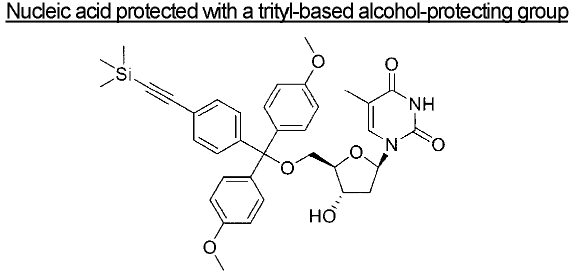

Pyridine (2.0 mL) was added to thymidine (nucleic acid) (90 mg, 0.37 mmol) and 4-trimethylsilylethynyl-4',4"-dimethoxyltrityl chloride (Compound 4 and a trityl-based alcohol protecting group in Schemes 1 and 2) (178 mg, 0.41 mmol), and the resulting mixture was stirred at 50°C overnight. The stirred mixture was then concentrated, and an aqueous ammonium chloride solution was added. The resulting mixture was extracted with ethyl acetate, and the organic layer was dried over magnesium sulfide and concentrated.

The residue was purified by silica gel column chromatography (hexane/ethyl acetate = 1/1 → 0/1) to obtain the target compound (90 mg, 0.14 mmol) as a white solid (a nucleic acid protected with a trityl-based alcohol protecting group).

¹H-NMR (DMSO-d₆): δ 11.33 (s, 1H), 7.45 (d, 1H, J = 1.2 Hz), 7.39-7.37 (m, 4H), 7.25-7.22 (m, 4H), 6.91-6.88 (m, 4H), 6.20 (t, 1H, J = 6.6 Hz), 5.30 (d, 1H, J = 4.5 Hz), 4.28-4.22 (m, 1H), 3.89-3.85 (m, 1H), 3.74 (s, 6H), 3.23-3.12 (m , 2H), 2.27-2.13 (m, 2H), 1.50 (d, 3H, J = 1.2 Hz), 0.21 (s, 9H)
¹³C-NMR (DMSO-d₆): δ 163.7, 158.3, 150.4, 145.8, 135.7, 134.9, 134.5, 131.2, 129.8, 129.7, 127.9, 120.6, 113.3 109.6, 104.9, 94.3, 85.6, 85.3, 83.8, 70.5, 64.0, 55.1, 39.3, 11.8, -0.1

### [2] Production 2 of trityl-based alcohol protecting group (Scheme 3)

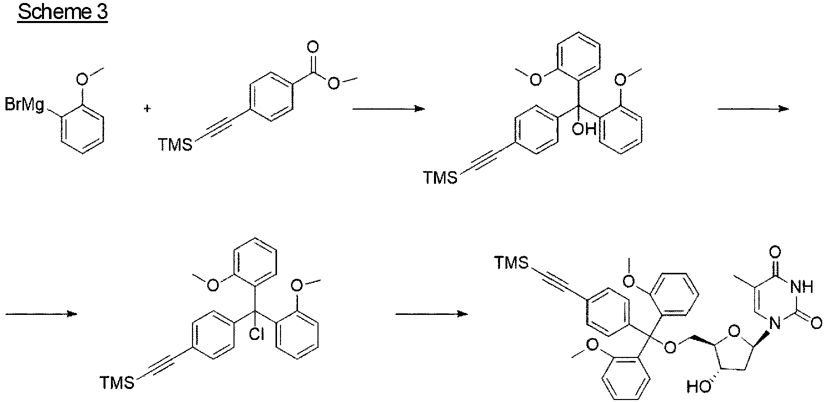

### (2-1) Production of 4-trimethylsilylethynyl-2',2"-dimethoxyltrityl alcohol

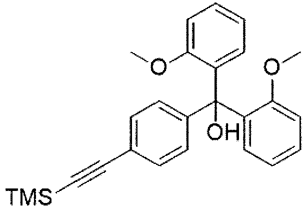

Under argon atmosphere, magnesium powder (306 mg, 12.6 mmol) and iodine (1 item) were placed in a 100 mL eggplant flask, and THF (10 mL) was added. The resulting mixture was stirred at room temperature. A solution (3.5 mL) of 2-bromoanisole (1.57 g, 8.40 mmol) in THF was added dropwise thereto, and the resulting mixture was stirred at a temperature of 40°C to 50°C for 1 hour.

Methyl 4-(trimethylsillylethynyl)benzoate (888 mg, 3.82 mmol) was added to this reaction liquid, and the resulting mixture was stirred at room temperature for 1 hour. After completion of the reaction was confirmed, 1N hydrochloric acid was added to the reaction liquid, and the resulting mixture was extracted with ethyl acetate. The organic layer was dried over magnesium sulfide and concentrated.

The residue was purified by silica gel column chromatography (hexane/ethyl acetate = 19/1 → 4/1) to obtain the target compound (1.45 g, 3.48 mmol) as a yellow solid.

¹H-NMR (CDCl₃): δ 7.38-7.34 (m, 2H), 7.32-7.26 (m, 2H), 7.22-7.18 (m, 2H), 7.00-6.97 (m, 2H), 6.93-6.85 (m, 4H), 5.26 (s, 1H), 3.52 (s, 6H), 0.24 (s, 9H)
¹³C-NMR (CDCl₃): δ 157.5, 147.7, 133.6, 131.1, 129.5, 129.0, 127.8, 121.1, 120.6, 112.4, 105.7, 93.7, 80.9, 55.6, 0.2

### (2-2) Production of 4-trimethylsilylethynyl-2',2"-dimethoxyltrityl chloride

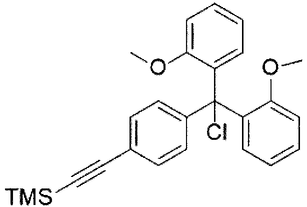

Ethyl acetate (2.4 mL) and thionyl chloride (0.35 mL, 4.8 mmol) were added to 4-trimethylsilylethynyl-2',2"-dimethoxyltrityl alcohol (0.20 g, 0.48 mmol), and the resulting mixture was stirred at 45°C for 4 hours. The stirred mixture was concentrated to obtain a residue of the target compound as a beige solid.

¹H-NMR (CDCl₃): δ 7.37-7.27 (m, 6H), 7.15-7.11 (m, 2H), 6.92-6.84 (m, 4H), 3.51 (s, 6H), 0.25 (s, 9H)
¹³C-NMR (CDCl₃): δ 157.7, 145.0, 132.0, 130.7, 130.1, 129.8, 129.2, 121.5, 120.1, 112.8, 105.3, 94.3, 80.9, 55.6, 0.1

### (2-3) Protection of nucleic acid by trityl-based alcohol protecting group

### 5'-O-(4-Trimethylsilylethynyl-2',2"-dimethoxyltrityl)-thymidine

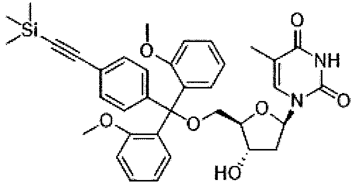

Pyridine (1.0 mL) was added to thymidine (67 mg, 0.28 mmol) and 4-trimethylsilylethynyl-2',2"-dimethoxyltrityl chloride (190 mg, 0.44 mmol), and the resulting mixture was stirred at 50°C for 3 hours and then concentrated. After adding water, the resulting mixture was extracted with ethyl acetate. The organic layer was dried over magnesium sulfide and concentrated.

The residue was purified by silica gel column chromatography (hexane/ethyl acetate 1/1 → 0/1) to obtain the target compound (21 mg, 0.033 mmol) as a white solid.

¹H-NMR (DMSO-d₆): δ 11.33 (s, 1H), 7.49 (d, 1H, J = 0.9 Hz), 7.43-7.39 (m, 1H), 7.35-7.24 (m, 7H), 7.01-6.90 (m, 4H), 6.20 (t, 1H, J = 6.6 Hz), 5.31 (d, 1H, J = 4.5 Hz), 4.38-4.33 (m, 1H), 3.91-3.88 (m, 1H), 3.40 (s, 3H), 3.38 (s, 3H)), 3.22 (dd, 1H, J = 10.5, 4.5 Hz), 3.13 (dd, 1H, J = 10.5, 2.7 Hz), 2.25-2.08 (m, 2H), 1.42 (d, 3H, J = 0.9 Hz), 0.19 (s, 9H)
¹³C-NMR (DMSO-d₆): δ 163.7, 157.2, 156.9, 150.4, 145.8, 135.8, 130.0, 129.7, 129.3, 129.3, 129.1, 128.2, 128.0, 120.2, 120.0, 119.5, 112.3, 112.2, 109.5, 105.5, 93.4, 85.5, 84.4, 83.8, 70.5, 64.5, 54.9, 54.8, 39.6, 11.8, -0.1

### [3] Production 3 of trityl-based alcohol protecting group (Scheme 4)

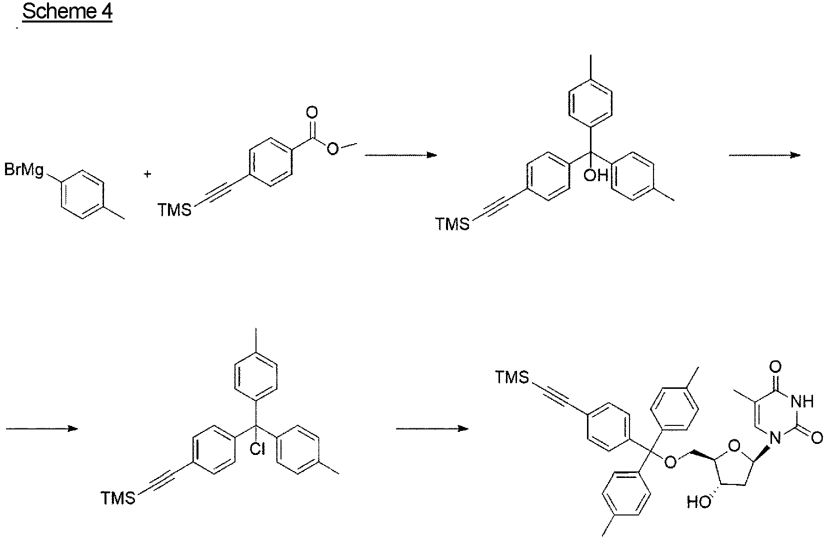

### (3-1) Production of 4-trimethylsilylethynyl-4',4"-dimethyltrityl alcohol

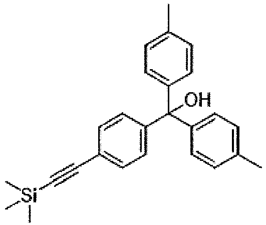

Under argon atmosphere, magnesium powder (237 mg, 9.77 mmol) and iodine (2 items) were placed in a 100 mL eggplant flask, and THF (10 mL) was added. The resulting mixture was stirred at room temperature. A solution (3.5 mL) of 4-bromotoluene (1.11 g, 6.51 mmol) in THF was added dropwise thereto, and the resulting mixture was stirred at 50°C for 1 hour.

Methyl 4-(trimethylsillylethynyl)benzoate (688 mg, 2.96 mmol) was added to this reaction liquid, and the resulting mixture was stirred at room temperature for 2.5 minutes. 1N HCl was then added to the reaction liquid, and the resulting mixture was extracted with ethyl acetate. The organic layer was dried over magnesium sulfide and concentrated.

The residue was purified by silica gel column chromatography (hexane/ethyl acetate = 98/2 → 95/5) to obtain the target compound (750 mg, 1.95 mmol) as a yellow solid.

¹H-NMR (CDCl₃): δ 7.42-7.38 (m, 2H), 7.26-7.22 (m, 2H), 7.11 (s, 8H), 2.69 (s, 1H), 2.34 (s, 6H), 0.24 (s, 9H)
¹³C-NMR (CDCl₃): δ 147.6, 143.9, 137.2, 131.6, 128.8, 127.9, 127.9, 122.0, 105.1, 94.5, 81.8, 21.2, 0.1

### (3-2) Production of 4-trimethylsilylethynyl-4',4"-dimethyltrityl chloride

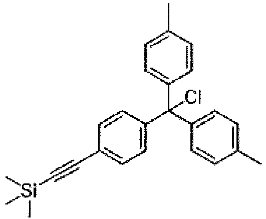

Thionyl chloride (0.16 mL, 2.2 mmol) was added to 4-trimethylsilylethynyl-4',4"-dimethyltrityl alcohol (170 mg, 0.44 mmol), and the resulting mixture was stirred at 45°C for 1.5 hours. The stirred mixture was then concentrated to give the target compound (177 mg, 0.44 mmol) as a white-yellow solid.

¹H-NMR (CDCl₃): δ 7.40-7.36 (m, 2H), 7.21-7.17 (m, 2H), 7.09 (s, 8H), 2.36 (s, 6H), 0.25 (s, 9H)
¹³C-NMR (CDCl₃): δ 146.0, 142.3, 137.9, 131.4, 129.7, 129.7, 128.6, 122.6, 104.7, 95.2, 81.3, 21.2, 0.1

### (3-3) Protection of nucleic acid using a trityl-based alcohol protecting group

### 5'-O-(4-Trimethylsilylethynyl-4',4"-dimethyltrityl)-thymidine

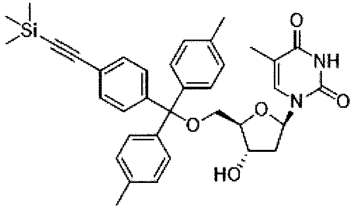

4-Trimethylsilylethynyl-4',4"-dimethyltrityl chloride (165 mg, 0.41 mmol) and thymidine (66 mg, 0.27 mmol) were dissolved in pyridine (1.0 mL), and the resulting mixture was stirred at 50°C overnight and then concentrated. The organic layer was extracted with ethyl acetate and dried over magnesium sulfide sulfate, and the solvent was distilled off.

The residue was purified by column chromatography (hexane/ethyl acetate = 1/1 → 0/1) to obtain the target compound (87.9 mg, 0.144 mmol) as a white solid.

¹H-NMR (DMSO-d₆): δ 11.33 (s, 1H), 7.45 (d, 1H, J = 1.2 Hz), 7.41-7.35 (m, 4H), 7.23-7.20 (m, 4H), 7.15-7.12 (m, 4H), 6.20 (t, 1H, J = 6.6 Hz), 5.30 (d, 1H, J = 4.5 Hz), 4.27-4.21 (m, 1H), 3.89-3.85 (m, 1H), 3.23-3.11 (m, 1H), 2.27 (s , 6H), 2.24-2.06 (m, 2H), 1.52 (d, 3H, J = 1.2 Hz), 0.21 (s, 9H)
¹³C-NMR (DMSO-d₆): δ 163.7, 150.4, 145.3, 140.3, 139.8, 136.5, 135.8, 131.2, 128.6, 128.4, 128.3, 128.2, 120.7, 109.6, 104.9, 94.4, 85.9, 85.3, 83.7, 70.4, 64.2, 39.5, 20.8, 11.8, -0.1

### [4] Production 4 of trityl-based alcohol protecting group (Scheme 5)

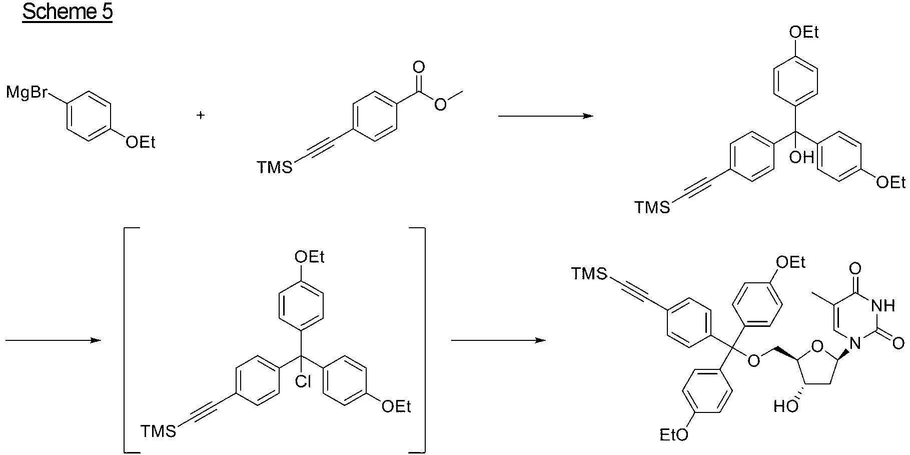

### (4-1) Production of 4-trimethylsilylethynyl-4',4"-diethoxyltrityl alcohol

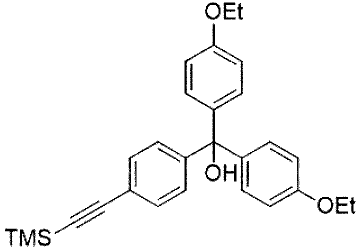

Under argon atmosphere, magnesium powder (237 mg, 9.77 mmol) and iodine (1 item) were placed in a 100 mL eggplant flask, and THF (10 mL) was added. The resulting mixture was stirred at room temperature. A solution (3.5 mL) of 4-bromophenetole (0.928 mL, 6.51 mmol) in THF was added dropwise thereto, and the resulting mixture was stirred at 50°C or lower for 1 hour.

Methyl 4-(trimethylsillylethynyl)benzoate (570 mg, 2.45 mmol) was added to this reaction liquid, and the resulting mixture was stirred at room temperature for 2.5 hours. 1N HCl was then added to the reaction liquid, and the resulting mixture was extracted with ethyl acetate. The organic layer was dried over magnesium sulfide and concentrated.

The residue was purified by silica gel column chromatography (hexane/ethyl acetate =98/2 → 95/5) to obtain the target compound (653 mg, 1.47 mmol) as a yellow oil.

¹H-NMR (CDCl₃): δ 7.42-7.38 (m, 2H), 7.26-7.21 (m, 2H), 7.14-7.09 (m, 4H), 6.83-6.78 (m, 4H), 4.02 (q, 4H, J = 6.9 Hz), 2.70 (s, 1H), 1.41 (t, 6H, J = 6.9 Hz), 0.24 (s, 9H)
¹³C-NMR (CDCl₃): δ 158.3, 147.8, 139.0, 131.6, 129.2, 127.8, 121.9, 113.9, 105.1, 94.4, 81.5, 63.6, 15.0, 0.1

### (4-2) Protection of nucleic acid using a trityl-based alcohol protecting group

### 5'-O-(4-Trimethylsilylethynyl-4',4"-diethoxyltrityl)-thymidine

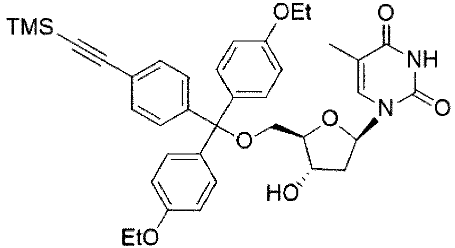

4-Trimethylsilylethynyl-4',4"-diethoxyltrityl alcohol (0.27 g, 0.60 mmol) was dissolved in ethyl acetate (3.0 mL), and thionyl chloride (0.22 mL, 3.0 mmol) was added. The resulting mixture was stirred at 45°C for 1.5 hours. The stirred mixture was then concentrated to give a product. Subsequently, this product was dissolved in pyridine (3.0 mL), and thymidine (73 mg, 0.30 mmol) was added. The resulting mixture was stirred at 45°C overnight.

The stirred mixture was concentrated, and the residue was purified by silica gel column chromatography (hexane/ethyl acetate = 1/1 → 0/1) to obtain the target compound (45 mg, 0.067 mmol) as a white solid.

¹H-NMR (DMSO-d₆): δ 11.32 (s, 1H), 7.45 (d, 1H, J = 0.9 Hz), 7.39-7.37 (m, 4H), 7.23-7.19 (m, 4H), 6.89-6.86 (m, 4H), 6.20 (t, 1H, J = 6.6 Hz), 5.30 (d, 1H, J = 4.5 Hz), 4.28-4.22 (m, 1H), 4.00 (q, 4H, J = 6.9 Hz), 3.89-3.85 (m, 1H), 3.22-3.12 (m, 2H), 2.27-2.08 (m, 2H), 1.50 (d, 3H, J = 0.9 Hz), 1.30 (t, 6H, J = 6.9 Hz), 0.21 (s, 9H)
¹³C-NMR (DMSO-d₆): δ 163.6, 157.6, 150.4, 145.8, 135.7, 134.8, 134.4, 131.2, 129.8, 129.7, 127.9, 120.6, 113.7, 109.5, 104.9, 94.3, 85.6, 85.3, 83.7, 70.4, 63.0, 39.4, 14.6, 11.8, -0.1

### [5] Production 5 of trityl-based alcohol protecting group (Scheme 6)

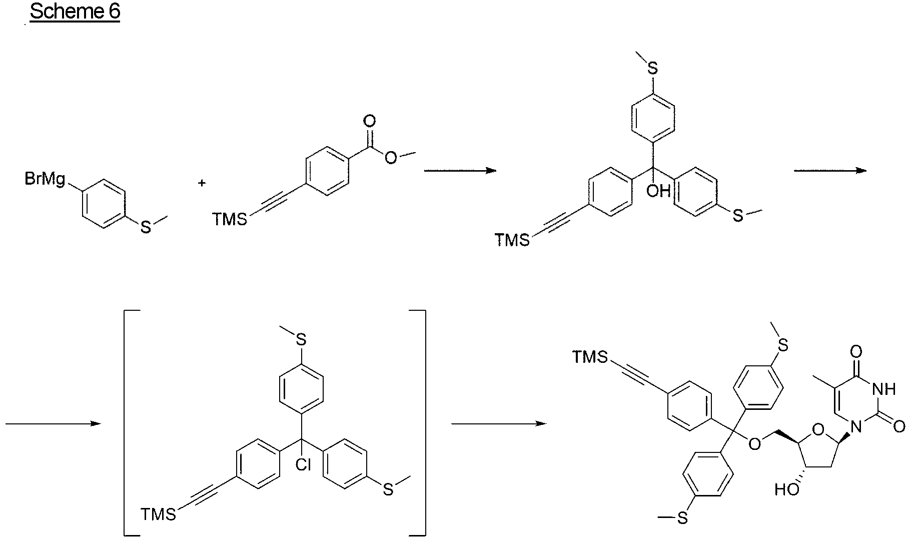

### (5-1) Production of 4-trimethylsilylethynyl-4',4"-dimethylthiotrityl alcohol

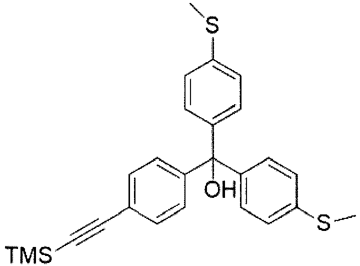

Under argon atmosphere, magnesium powder (237 mg, 9.77 mmol) and iodine (1 piece) were placed in a 100 mL eggplant flask, and THF (10 mL) was added. The resulting mixture was stirred at room temperature. A solution (3.5 mL) of 4-bromothioanisole (1.32 g, 6.51 mmol) in THF was added dropwise thereto, and the resulting mixture was stirred at 50°C or lower for 1 hour.

Methyl 4-(trimethylsillylethynyl)benzoate (690 mg, 2.96 mmol) was added to this reaction liquid, and the resulting mixture was stirred at room temperature overnight. 1N HCl was then added to the reaction liquid, the resulting mixture was extracted with ethyl acetate. The organic layer was dried over magnesium sulfide and concentrated.

The residue was purified by silica gel column chromatography (hexane/ethyl acetate = 98/2 → 95/5) to obtain the target compound (1.03 g, 2.29 mmol).

¹H-NMR (CDCl₃): δ 7.43-7.38 (m, 2H), 7.24-7.12 (m, 10H), 2.69 (s, 1H), 2.47 (s, 6H), 0.24 (s, 9H)
¹³C-NMR (CDCl₃): δ 147.0, 143.3, 138.0, 131.7, 128.4, 127.8, 126.1, 122.3, 104.9, 94.8, 81.5, 15.8, 0.1

### (5-2) Protection of nucleic acid using a trityl-based alcohol protecting group

### 5'-O-(4-Trimethylsilylethynyl-4',4"-dimethylthiotrityl)-thymidine

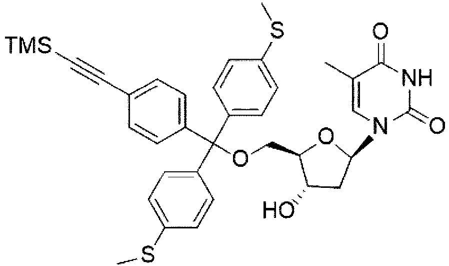

4-Trimethylsilylethynyl-4',4"-dimethylthiotrityl alcohol (0.30 g, 0.67 mmol) was dissolved in ethyl acetate (3.2 mL), and thionyl chloride (0.24 mL, 3.4 mmol) was added. The resulting mixture was stirred at 45°C for 1.5 hour and then concentrated to obtain a product. Subsequently, this product was dissolved in pyridine (3.0 mL), and thymidine (73 mg, 0.30 mmol) was added. The resulting mixture was stirred at 45°C overnight.

The stirred mixture was concentrated, and the residue was purified by silica gel column chromatography (hexane/ethyl acetate =1/1 → 0/1) to obtain the target compound (115 mg, 0.17 mmol) as a white solid.

¹H-NMR (DMSO-d₆): δ 11.33 (s, 1H), 7.44 (d, 1H, J = 1.2 Hz), 7.43-7.36 (m, 4H), 7.29-7.20 (m, 8H), 6.20 (t , 1H, J = 6.6 Hz), 5.30 (d, 1H, J = 4.8 Hz), 4.28-4.21 (m, 1H), 3.90-3.86 (m, 1H), 3.22 (dd, 1H, J = 5.4, 7.5 Hz), 3.13 (dd, 1H, J = 3.0, 7.5 Hz), 2.45 (s, 6H), 2.28-2.19 (m, 1H), 2.16-2.08 (m, 1H), 1.54 (d, 3H, J = 1.2 Hz), 0.21 (s, 9H)
¹³C-NMR (DMSO-d₆): δ 163.6, 150.4, 144.7, 139.3, 139.0, 137.5, 135.8, 131.3, 128.9, 128.8, 128.1, 125.3, 120.9, 109.6, 104.8, 94.5, 85.6, 85.2, 83.7, 70.3, 64.2, 39.4, 14.1, 11.8, -0.1

### Industrial Applicability

The trityl-based alcohol protecting group of the present invention comprises a DMTr or like protecting group and at least one triple bond introduced into the protecting group. The trityl-based alcohol protecting group of the present invention is a novel trityl-based alcohol protecting group that is useful in synthesizing nucleic acids. The trityl-based alcohol protecting group of the compound represented by formula (1) of the present invention is excellent in controlling the ease of deprotection.

In the trityl-based alcohol protecting group of the compound represented by formula (1) of the present invention, a protecting group, such as a DMTr protecting group, changes in stability of the cation and forms a trityl-based alcohol protecting group that is easily or instantly removed, for example, under weak acidic conditions. The trityl-based alcohol protecting group of the compound represented by formula (1) of the present invention is excellent in that the triple bond introduced is detectable by Raman spectroscopy and various reactions can be tracked based on the trityl-based alcohol protecting group.

## Claims

1. A compound represented by the following formula (1): 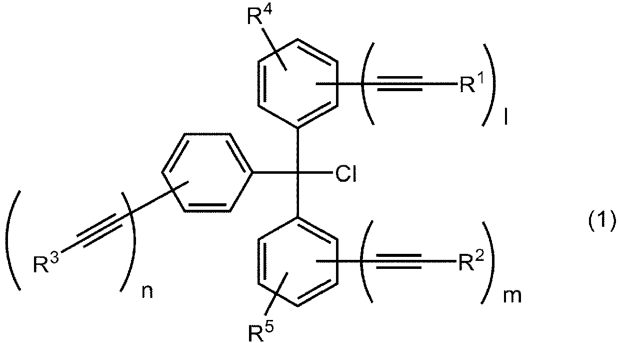 wherein
R¹, R², and R³ are same or different and each represent a hydrogen atom, an alkyl group, a trimethylsilyl group, an acetyl group, a benzoyl group, an ester group, an alkyl ester group, an aryl group, an alkyl alcohol group, a nitrile group, a group represented by 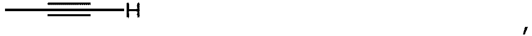 or a group represented by 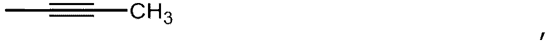
l, m, and n each represent an integer of 0, 1, or 2, provided that l, m, and n are not 0 at the same time, and
R⁴ and R⁵ are the same or different and each represent an electron-donating group:
the electron-donating group is:
a linear or branched alkyl group, such as methyl, ethyl, n-propyl, isopropyl, n-butyl, isobutyl, or tert-butyl;
a cycloalkyl group, such as cyclopropyl, cyclobutyl, or cyclopentyl;
an alkenyl group, such as vinyl, allyl, or 1-propenyl;
a cycloalkenyl group, such as 1-cyclopentenyl or 1-cyclohexenyl;
an aryl group, such as phenyl;
an aralkyl group, such as benzyl or phenethyl;
an alkoxy group, such as methoxy, ethoxy, n-propoxy, iso-propoxy, n-butoxy, or tert-butoxy;
an aryloxy group, such as phenyloxy;
a hydroxy group;
an amino group;
an amino group having one or two alkyl groups, such as methylamino, ethylamino, n-propylamino, dimethylamino, or diethylamino;
an amino group having one or two aryl groups, such as phenylamino; or
a hydroxyamino group;
an alkyl sulfide group, such as a methyl sulfide group, an ethyl sulfide group, an n-propyl sulfide group, an isopropyl sulfide group, an n-butyl sulfide group, an isobutyl sulfide group, or a tert-butyl sulfide group.

2. The compound according to claim 1, wherein the electron-donating group is a linear or branched alkyl group, an alkoxy group, a hydroxy group, an amino group, or an amino group having one or two alkyl group.

3. The compound according to claim 1, wherein the electron-donating group is an alkoxy group, a hydroxy group, or an amino group.

4. The compound according to claim 1, wherein the electron-donating group is an alkoxy group having 1 to 4 carbon atoms.

5. The compound according to claim 1, wherein the electron-donating group is an alkoxy group, such as methoxy, ethoxy, n-propoxy, isopropoxy, n-butoxy, or tert-butoxy.

6. The compound according to claim 1, wherein the electron-donating group is a linear or branched alkyl group, such as methyl, ethyl, n-propyl, isopropyl, n-butyl, isobutyl, or tert-butyl.

7. The compound according to claim 1, wherein the electron-donating group is an alkylsulfide group, such as a methylsulfide group or an ethylsulfide group.

## Patentansprüche

1. Verbindung, dargestellt durch die folgende Formel (1): 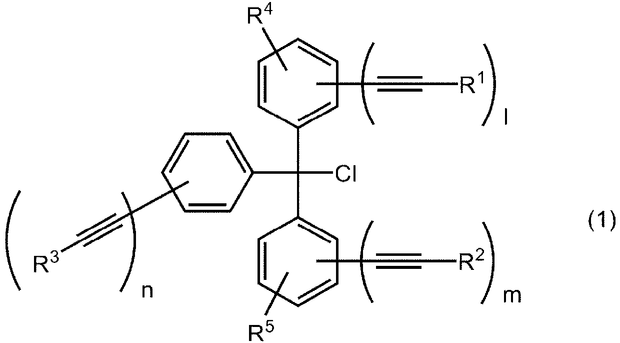 wobei
R¹, R² und R³ gleich oder unterschiedlich sind und jeweils ein Wasserstoffatom, eine Alkylgruppe, eine Trimethylsilylgruppe, eine Acetylgruppe, eine Benzoylgruppe, eine Estergruppe, eine Alkylestergruppe, eine Arylgruppe, eine Alkylalkoholgruppe, eine Nitrilgruppe, eine Gruppe dargestellt durch 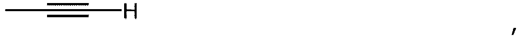 oder eine Gruppe dargestellt durch 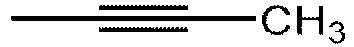 darstellen,
l, m und n jeweils eine ganze Zahl von 0, 1 oder 2 darstellen, vorausgesetzt, dass l, m und n nicht gleichzeitig 0 sind, und
R⁴ und R⁵ gleich oder unterschiedlich sind und jeweils eine Elektronendonorgruppe darstellen:
die Elektronendonorgruppe ist:
eine lineare oder verzweigte Alkylgruppe, wie etwa Methyl, Ethyl, n-Propyl, Isopropyl, n-Butyl, Isobutyl oder tert-Butyl;
eine Cycloalkylgruppe, wie etwa Cyclopropyl, Cyclobutyl oder Cyclopentyl;
eine Alkenylgruppe, wie etwa Vinyl, Allyl oder 1-Propenyl;
eine Cycloalkenylgruppe, wie etwa 1-Cyclopentenyl oder 1-Cyclohexenyl;
eine Arylgruppe, wie etwa Phenyl;
eine Aralkylgruppe, wie etwa Benzyl oder Phenethyl;
eine Alkoxygruppe, wie etwa Methoxy, Ethoxy, n-Propoxy, Iso-Propoxy, n-Butoxy oder tert-Butoxy;
eine Aryloxygruppe, wie etwa Phenyloxy;
eine Hydroxygruppe;
eine Aminogruppe;
eine Aminogruppe mit einer oder zwei Alkylgruppen, wie etwa Methylamino, Ethylamino, n-Propylamino, Dimethylamino oder Diethylamino;
eine Aminogruppe mit einer oder zwei Arylgruppen, wie etwa Phenylamino; oder
eine Hydroxyaminogruppe;
eine Alkylsulfidgruppe, wie etwa eine Methylsulfidgruppe, eine Ethylsulfidgruppe, eine n-Propylsulfidgruppe, eine Isopropylsulfidgruppe, eine n-Butylsulfidgruppe, eine Isobutylsulfidgruppe oder eine tert-Butylsulfidgruppe.

2. Verbindung nach Anspruch 1, wobei die Elektronendonorgruppe eine lineare oder verzweigte Alkylgruppe, eine Alkoxygruppe, eine Hydroxygruppe, eine Aminogruppe oder eine Aminogruppe mit einer oder zwei Alkylgruppen ist.

3. Verbindung nach Anspruch 1, wobei die Elektronendonorgruppe eine Alkoxygruppe, eine Hydroxygruppe oder eine Aminogruppe ist.

4. Verbindung nach Anspruch 1, wobei die Elektronendonorgruppe eine Alkoxygruppe mit 1 bis 4 Kohlenstoffatomen ist.

5. Verbindung nach Anspruch 1, wobei die Elektronendonorgruppe eine Alkoxygruppe ist, wie etwa Methoxy, Ethoxy, n-Propoxy, Isopropoxy, n-Butoxy oder tert-Butoxy.

6. Verbindung nach Anspruch 1, wobei die Elektronendonorgruppe eine lineare oder verzweigte Alkylgruppe ist, wie etwa Methyl, Ethyl, n-Propyl, Isopropyl, n-Butyl, Isobutyl oder tert-Butyl.

7. Verbindung nach Anspruch 1, wobei die Elektronendonorgruppe eine Alkylsulfidgruppe ist, wie etwa eine Methylsulfidgruppe oder eine Ethylsulfidgruppe.

## Revendications

1. Composé représenté par la formule suivante (1) : 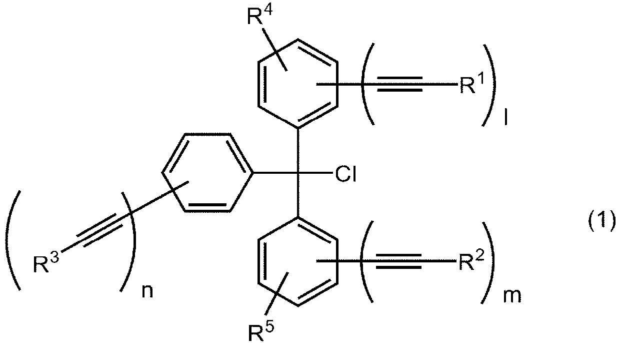 dans lequel
R¹, R² et R³ sont identiques ou différents et représentent chacun un atome d'hydrogène, un groupe alkyle, un groupe triméthylsilyle, un groupe acétyle, un groupe benzoyle, un groupe ester, un groupe ester d'alkyle, un groupe aryle, un groupe alcool d'alkyle, un groupe nitrile, un groupe représenté par 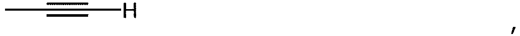 ou un groupe représenté par 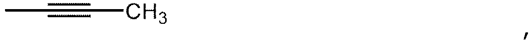
l, m et n représentent chacun un nombre entier de 0, 1 ou 2, à condition que l, m et n ne soient pas simultanément égaux à 0, et
R⁴ et R⁵ sont identiques ou différents et représentent chacun un groupe donneur d'électrons :
le groupe donneur d'électrons est :
un groupe alkyle linéaire ou ramifié, tel que méthyle, éthyle, n-propyle, isopropyle, n-butyle, isobutyle ou tert-butyle ;
un groupe cycloalkyle, tel que cyclopropyle, cyclobutyle ou cyclopentyle ;
un groupe alcényle, tel que vinyle, allyle ou 1-propényle ;
un groupe cycloalcényle, tel que 1-cyclopentényle ou 1-cyclohexényle ;
un groupe aryle, tel que phényle ;
un groupe aralkyle, tel que benzyle ou phénéthyle ;
un groupe alcoxy, tel que méthoxy, éthoxy, n-propoxy, isopropoxy, n-butoxy ou tert-butoxy ;
un groupe aryloxy, tel que phényloxy ;
un groupe hydroxy ;
un groupe amino ;
un groupe amino comportant un ou deux groupes alkyle, tel que méthylamino, éthylamino, n-propylamino, diméthylamino ou diéthylamino ;
un groupe amino comportant un ou deux groupes aryle, tel que phénylamino ; ou
un groupe hydroxyamino ;
un groupe sulfure d'alkyle, tel qu'un groupe sulfure de méthyle, un groupe sulfure d'éthyle, un groupe sulfure de n-propyle, un groupe sulfure d'isopropyle, un groupe sulfure de n-butyle, un groupe sulfure d'isobutyle ou un groupe sulfure de tert-butyle.

2. Composé selon la revendication 1, dans lequel le groupe donneur d'électrons est un groupe alkyle linéaire ou ramifié, un groupe alcoxy, un groupe hydroxy, un groupe amino ou un groupe amino comportant un ou deux groupes alkyle.

3. Composé selon la revendication 1, dans lequel le groupe donneur d'électrons est un groupe alcoxy, un groupe hydroxy ou un groupe amino.

4. Composé selon la revendication 1, dans lequel le groupe donneur d'électrons est un groupe alcoxy comportant de 1 à 4 atomes de carbone.

5. Composé selon la revendication 1, dans lequel le groupe donneur d'électrons est un groupe alcoxy, tel que méthoxy, éthoxy, n-propoxy, isopropoxy, n-butoxy ou tert-butoxy.

6. Composé selon la revendication 1, dans lequel le groupe donneur d'électrons est un groupe alkyle linéaire ou ramifié, tel que méthyle, éthyle, n-propyle, isopropyle, n-butyle, isobutyle ou tert-butyle.

7. Composé selon la revendication 1, dans lequel le groupe donneur d'électrons est un groupe sulfure d'alkyle, tel qu'un groupe sulfure de méthyle ou un groupe sulfure d'éthyle.
